# EUROPEAN PATENT SPECIFICATION

(11) **EP 4 592 402 B1**
(45) Date of publication and mention of the grant of the patent: **11.11.2026**
(21) Application number: 25180886.1
(22) Date of filing: 29.06.2022
(51) Int. Cl.: C12Q 1/6806, C12Q 1/6851

(54) **ENZYME FORMULATIONS AND REACTION MIXTURES FOR NUCLEIC ACID AMPLIFICATION**
ENZYMFORMULIERUNGEN UND REAKTIONSMISCHUNGEN ZUR NUKLEINSÄUREAMPLIFIKATION
FORMULATIONS ENZYMATIQUES ET MÉLANGES RÉACTIONNELS POUR L'AMPLIFICATION D'ACIDES NUCLÉIQUES

(30) Priority: 01.07.2021 US 202163217560 P
(43) Date of publication of application: 30.07.2025
(62) Document number(s) of the earlier application(s) in accordance with Art. 76 EPC: 22747213.1 / 4 363 610
(73) Proprietor: Gen-Probe Incorporated, San Diego, CA 92121 (US)
(72) Inventor: FILIPOWSKY, Mark E., San Marcos, CA 92078 (US); CHABOT, Vincent J., Escondido, CA 92026 (US)
(74) Representative: Forresters IP LLP

(56) References cited:
- WO-A1-2022/198086
- US-A1- 2011 256 592

## Description

### REFERENCE TO SEQUENCE LISTING

The instant application contains a Sequence Listing which has been submitted in ASCII format via EFS-Web. Said ASCII Copy, created on June 8, 2022, is named "GPR_7910PC_20220608_Seq_Listing_ST25" and is 4,974 bytes in size.

### BACKGROUND

Nucleic acid amplification methods are widely used in many bioscience applications, including, for example, *in vitro* detection assays. Methods for amplification of target nucleic acids from biological samples typically employ sample extraction step(s) that leave one or more residual detergents, which are often inhibitory to polymerase enzymes, in the extracted sample. The inhibitory effect of such residual detergents presents challenges to the formulation of polymerase enzymes and amplification reaction mixtures.
WO 2022/198086 A1 discloses methods, compositions, and kits for use in detecting a target nucleic acid sequence in a sample.
US 2011/256592 A1 is directed to methods for in vitro DNA synthesis catalysed by a DNA polymerase using cyclodextrins.

### SUMMARY

The invention is defined in the appended claims. Any statements described herein forming part of the present disclosure but falling outside the scope of the appended claims do not form part of the claimed invention. In one aspect, the present disclosure provides an aqueous polymerase formulation. The formulation generally includes at least one polymerase, α-cyclodextrin at a concentration of from 10 mg/mL to 40 mg/mL, polysorbate 20 at a concentration of from 0.002% to 0.05% (v/v), and at least one buffering agent. The formulation further includes a lyoprotectant. A particularly suitable lyoprotectant is trehalose, which may be present, for example at a concentration of from 0.2 M to 0.4 M.

In another aspect, the present disclosure provides a method of preparing a lyophilized polymerase formulation. The method generally includes (a) providing an aqueous formulation as above, and (b) lyophilizing the aqueous formulation to form the lyophilized polymerase formulation. In another aspect, the present invention provides a lyophilized polymerase formulation prepared by the foregoing method.

In another aspect, the present disclosure provides a lyophilized polymerase formulation that enables reconstitution into an aqueous formulation as set forth above.

In another aspect, the present disclosure provides a method of preparing an aqueous polymerase formulation. The method generally includes (a) providing a lyophilized polymerase formulation as above, and (b) dissolving the lyophilized polymerase formulation in a diluent to provide a reconstituted formulation.

In another aspect, the present invention provides a kit having a first sealed container containing a lyophilized polymerase formulation that enables reconstitution into an aqueous polymerase formulation comprising: at least one polymerase; α-cyclodextrin at a concentration of from 10 mg/mL to 40 mg/mL; polysorbate 20 at a concentration of from 0.002% to 0.05% (v/v); at least one buffering agent; and a lyoprotectant. In some embodiments, the kit further includes a second sealed container containing a diluent.

In another aspect, the present disclosure provides an amplification reaction mixture. The reaction mixture may generally include at least one polymerase, α-cyclodextrin at a concentration of from 5 mg/mL to 20 mg/mL, polysorbate 20 at a concentration of from 0.001% to 0.025% (v/v), and at least one buffering agent. In some variations, the reaction mixture may further include nucleotide triphosphates suitable for performing nucleic acid amplification. The reaction mixture may include at least one amplification oligomer configured to amplify a target region of a target nucleic acid, and/or at least one detection probe oligomer configured to specifically hybridize to a target sequence contained within the target region. The reaction mixture may further include the target nucleic acid to be amplified and/or at least one detergent such as, for example, an anionic detergent. Particularly suitable anionic detergents include sodium dodecyl sulfate (SDS) and lithium lauryl sulfate (LLS).

In another aspect, the present disclosure provides a method of preparing an amplification reaction mixture. The method may generally include (a) providing an aqueous polymerase formulation as above, and (b) mixing the aqueous formulation with a second formulation comprising at least one amplification oligomer configured to amplify a target region of a target nucleic acid; in some such variations, the method may further include mixing the mixture generated at step (b) with a sample containing or suspected of containing the target nucleic acid. The method may generally include (a) providing a lyophilized polymerase formulation as above, (b) dissolving the lyophilized polymerase formulation in a diluent to provide a reconstituted formulation, and (c) mixing the reconstituted formulation with a second formulation comprising at least one amplification oligomer configured to amplify a target region of a target nucleic acid; in some such variations, the method may further include mixing the mixture generated at step (c) with a sample containing or suspected of containing the target nucleic acid. The sample may be an extracted sample containing at least one detergent such as, for example, an anionic detergent. Particularly suitable anionic detergents include sodium dodecyl sulfate (SDS) and lithium lauryl sulfate (LLS). The sample containing or suspected of containing the target nucleic acid may constitute at least 20% or at least 40% of the reaction mixture.

In another aspect, the present disclosure provides a method for performing an amplification reaction. The method may generally include (a) providing a sample containing or suspected of containing a target nucleic acid; (b) contacting the sample with an aqueous mixture comprising at least one polymerase, α-cyclodextrin, polysorbate 20, at least one buffering agent, nucleotide triphosphates suitable for performing nucleic acid amplification, at least one cofactor, and at least one amplification oligomer configured to amplify a target region of the target nucleic acid, wherein contacting the sample with the aqueous mixture provides a reaction mixture in which the α-cyclodextrin is present at a concentration of from 5 mg/mL to 20 mg/mL and the polysorbate 20 is present at a concentration of from 0.001% to 0.025% (v/v); and (c) using the reaction mixture to perform an *in vitro* nucleic acid amplification reaction, wherein the target nucleic acid, if present in the sample, is used as a template for generating one or more amplicons corresponding to the target region. The method may further include detecting the one or more amplicons (e.g., detecting the one or more amplicons in real time); in some such variations, the detecting step may comprise contacting the *in vitro* nucleic acid amplification reaction with at least one detection probe oligomer configured to specifically hybridize to a target sequence contained within the one or more amplicons. The sample may be an extracted sample containing at least one detergent such as, for example, an anionic detergent (e.g., sodium dodecyl sulfate (SDS) or lithium lauryl sulfate (LLS)). In other, the sample may constitute at least 20% or at least 40% of the reaction mixture generated as step (b).

### DEFINITIONS

Unless defined otherwise, all technical and scientific terms used herein have the same meaning as commonly understood by one of ordinary skill in the art pertinent to the methods and compositions described. As used herein, the following terms and phrases have the meanings ascribed to them unless specified otherwise.

The terms "a," "an," and "the" include plural referents, unless the context clearly indicates otherwise.

"Sample" includes any specimen that may contain a target nucleic acid. Samples include "biological samples," which include any tissue or material derived from a living or dead human. The biological sample may be treated to physically or mechanically disrupt tissue or cell structure, thus releasing intracellular components into a solution which may further contain enzymes, buffers, salts, detergents, and the like, which are used to prepare a biological sample for analysis. Also, samples may include processed samples such as samples in which one or more components have been concentrated or purified. Processed samples include, *e.g*., those obtained from passing samples over or through a filtering device, or following centrifugation, or by adherence to a medium, matrix, or support.

A "detergent" refers to a substance that can disperse a hydrophobic substance (e.g., lipids) in water by emulsification and which can be used to lyse or solubilize a biological sample for subsequent analysis. Detergents may be ionic or nonionic.

The term "lyoprotectant" as used herein refers to a molecule that prevents or reduces chemical and/or physical instability of a protein or other substance upon lyophilization and subsequent storage. Exemplary lyoprotectants include sugars such as sucrose or trehalose; an amino acid such as monosodium glutamate or histidine; a methylamine such as betaine; a lyotropic salt such as magnesium sulfate; a polyol such as trihydric or higher sugar alcohols such as, *e.g*., glycerin, erythritol, glycerol, arabitol, xylitol, sorbitol, and mannitol propylene glycol, polyethylene glycol; Pluronics; and combinations thereof. In some embodiments, a lyoprotectant is a non-reducing sugar, such as trehalose or sucrose. When used in the context of an aqueous formulation or reaction mixture comprising α-cyclodextrin and polysorbate 20 as described herein, the term "lyoprotectant" refers to a molecule other than α-cyclodextrin, although α-cyclodextrin may still act as a secondary lyoprotectant.

As used herein, the term "polymerase" means any enzyme that can catalyze the polymerization of nucleotides (including analogs thereof) into a nucleic acid strand. Typically, such nucleotide polymerization occurs in a template-dependent fashion. Such polymerases can include, for example, naturally occurring polymerases and any subunits and truncations thereof, mutant polymerases, variant polymerases, recombinant, fusion or otherwise engineered polymerases, chemically modified polymerases, synthetic molecules or assemblies, and any analogs, homologs, derivatives, or fragments thereof that retain the ability to catalyze such polymerization. Optionally, the polymerase can be a mutant polymerase comprising one or more mutations involving the replacement of one or more amino acids with other amino acids, the insertion or deletion of one or more amino acids from the polymerase, or the linkage of parts of two or more polymerases, including linking two or more parts from different species or families of polymerases. Exemplary polymerases include DNA polymerases such as, *e.g.,* Taq polymerase or reverse transcriptases. In other variations, a polymerase is an RNA polymerase.

The term "cofactor" refers to a divalent cation, or a salt thereof, required for a polymerase enzymatic activity. Suitable cofactors for use with polymerases are generally known in the art and include, for example, magnesium (Mg²⁺) and manganese (Mn²⁺). Typical divalent cation salts for use in polymerase reaction mixtures include chloride salts (e.g., magnesium chloride, manganese chloride).

A "nucleotide" as used herein is a subunit of a nucleic acid consisting of a phosphate group, a 5-carbon sugar, and a nitrogenous base (also referred to herein as "nucleobase"). The 5-carbon sugar found in RNA is ribose. In DNA, the 5-carbon sugar is 2'-deoxyribose.

"Nucleic acid" and "polynucleotide" refer to a multimeric compound comprising nucleotides and/or nucleotide analogs linked together to form a biopolymer. The biopolymers include conventional RNA, conventional DNA, mixed RNA-DNA, and nucleotide analog containing versions thereof. A nucleic acid "backbone" may be made up of a variety of linkages, including one or more of sugar-phosphodiester linkages, peptide-nucleic acid bonds ("peptide nucleic acids" or PNA), phosphorothioate linkages, methylphosphonate linkages, or combinations thereof. Sugar moieties of a nucleic acid may be ribose, deoxyribose, or similar compounds with substitutions, *e.g*., analogs with a methoxy, fluoro or halide group at the 2' position of the ribose (also referred to herein as "2'-O-Me" or "2'-methoxy" or 2'-fluoro, or "2'-halide"). Nitrogenous bases may be conventional bases, adenine (A), uracil (U), guanine (G), thymine (T), and cytosine (C), and analogs thereof (e.g., inosine, 5 methyl 2' deoyxcytosine ("5-Me-dC" or "5MeC"), and isoguanine). Nucleic acids may include one or more "abasic" residues where the backbone includes no nitrogenous base for position(s) of the polymer.

"Oligomer," "oligonucleotide," or "oligo" refers to a nucleic acid of generally less than 1,000 nucleotides (nt), including those in a size range having a lower limit of about 5 nt and an upper limit of about 900 nt. The term oligonucleotide does not denote any particular function to the reagent; rather, it is used generically to cover all such reagents described herein. Oligomers may be referred to by a functional name (e.g., capture probe, detection probe, primer, or promoter primer) but those skilled in the art will understand that such terms refer to oligomers.

A "target nucleic acid" as used herein is a nucleic acid comprising a target sequence to be amplified. Target nucleic acids may be DNA or RNA and may be either single-stranded or doublestranded. The target nucleic acid may include other sequences besides the target sequence, which may not be amplified.

The term "target sequence" or "target nucleic acid sequence" as used herein refers to the particular nucleotide sequence of the target nucleic acid that is to be amplified and/or detected. The "target sequence" includes the complexing sequences to which oligonucleotides (e.g., priming oligonucleotides and/or promoter oligonucleotides) complex during an amplification processes *(e.g.,* PCR, TMA). Unless the context clearly dictates otherwise, where the target nucleic acid is originally single-stranded, the term "target sequence" will also refer to the sequence complementary to the "target sequence" as present in the target nucleic acid, and where the target nucleic acid is originally doublestranded, the term "target sequence" refers to both the sense (+) and antisense (-) strands.

"Nucleic acid amplification" refers to any well-known *in vitro* procedure that produces multiple copies of a target nucleic acid sequence. Examples of such procedures include transcription-associated methods, *e.g*., transcription-mediated amplification (TMA), nucleic acid sequence-based amplification (NASBA) and others (*e.g.*, US Patent Nos. 5,399,491, 5,554,516, 5,437,990, 5,130,238, 4,868,105, and 5,124,246), replicase-mediated amplification (*e.g.*, US Patent No. 4,786,600), polymerase chain reaction (PCR) (*e.g.,* US Patent Nos. 4,683,195, 4,683,202, and 4,800,159), ligase chain reaction (LCR) (*e.g.,* EP Patent No. 0320308), and strand-displacement amplification (SDA) (*e.g.,* US Patent No. 5,422,252).

By "amplicon" or "amplification product" is meant a nucleic acid molecule generated in a nucleic acid amplification reaction and which is derived from a target nucleic acid. An amplicon or amplification product contains a target nucleic acid sequence that may be of the same or opposite sense as the target nucleic acid.

An "amplification oligonucleotide" or "amplification oligomer" is an oligonucleotide that hybridizes to a target nucleic acid and participates in a nucleic acid amplification reaction, *e.g*., serving as a primer. Amplification oligomers can have 3' ends that are extended by polymerization as part of the nucleic acid amplification reaction. Amplification oligomers can alternatively have 3' ends that are not extended by polymerization, but provide a component that facilitates nucleic acid amplification, *e.g.,* a promoter sequence joined 5' to the target-specific sequence of the amplification oligomer. Such an amplification oligomer is referred to as a promoter provider. Amplification oligomers that provide both a 3' target-specific sequence that is extendable by polymerization and a 5' promoter sequence are referred to as promoter primers. Amplification oligomers may be optionally modified to include 5' non-target-specific sequences such as tags, promoters (as mentioned), or other sequences used or useful for manipulating or amplifying the primer or target oligonucleotide.

"Detection probe oligomer," "detection probe," or "probe" refers to an oligomer that hybridizes specifically to a target sequence, including an amplified product, under conditions that promote nucleic acid hybridization, for detection of the target nucleic acid. Detection may either be direct (*i.e.,* probe hybridized directly to the target) or indirect (*i.e.,* a probe hybridized to an intermediate structure that links the probe to the target). A probe's target-specific sequence generally refers to the specific sequence within a larger sequence which the probe hybridizes specifically. A detection probe may include target-specific sequence(s) and non-target-specific sequence(s). Such non-target-specific sequences can include sequences which will confer a desired secondary or tertiary structure, such as a hairpin structure, which can be used to facilitate detection and/or amplification.

"Label" or "detectable label" refers to a moiety or compound joined directly or indirectly to a probe that is detected or leads to a detectable signal. Direct joining may use covalent bonds or non-covalent interactions (*e.g.*, hydrogen bonding, hydrophobic or ionic interactions, and chelate or coordination complex formation) whereas indirect joining may use a bridging moiety or linker (*e.g.,* via an antibody or additional oligonucleotide(s), which amplify a detectable signal). Any detectable moiety may be used, *e.g*., radionuclide, ligand such as biotin or avidin, enzyme, enzyme substrate, reactive group, chromophore such as a dye or particle (*e.g.,* latex or metal bead) that imparts a detectable color, luminescent compound (*e.g*., bioluminescent, phosphorescent, or chemiluminescent compound such as an acridinium ester ("AE") compound), and fluorescent compound (*i.e.,* fluorophore). Fluorophores may be used in combination with a quencher molecule that absorbs light when in close proximity to the fluorophore to diminish background fluorescence. Detectably labeled probes include, for example, hydrolysis (*e.g.*, TaqMan^{™}) probes, AE-labeled probes, molecular torches, and molecular beacons.

The term "configured to" denotes an actual arrangement of the polynucleotide sequence configuration of a referenced oligonucleotide target-hybridizing sequence. For example, amplification oligomers that are configured to generate a specified amplicon from a target sequence have polynucleotide sequences that hybridize to the target sequence and can be used in an amplification reaction to generate the amplicon. Also, as an example, oligonucleotides that are configured to specifically hybridize to a target sequence have a polynucleotide sequence that specifically hybridizes to the referenced sequence under stringent hybridization conditions.

The term "diluent" as used herein refers to a solution suitable for altering or achieving an exemplary or appropriate concentration or concentrations as described herein.

The term "container" refers to something into which an object or liquid can be placed or contained, *e.g*., for storage (for example, a holder, receptacle, vessel, or the like).

Reference to a numerical range herein (*e.g*., "X to Y" or "from X to Y") includes the endpoints defining the range and all values falling within the range.

Unless otherwise apparent from the context, when a value is expressed as "about" X or "approximately" X, the stated value of X will be understood to be accurate to ±10%.

### DESCRIPTION

The present disclosure provides aqueous polymerase formulations and reaction mixtures, including related methods for preparing and using such formulations and reaction mixtures, comprising a combination of α-cyclodextrin and polysorbate 20. The formulations and reaction mixtures are based, in part, on the surprising observation that α-cyclodextrin and polysorbate 20 provide a synergistic effect in mitigating the inhibition of polymerase activity observed in the presence of detergents often used in sample extraction buffers (*e.g*., detergents such as sodium dodecyl sulfate (SDS) or lithium lauryl sulfate (LLS)). In certain aspects, this mitigating effect on polymerase inhibition provides advantages such as, *e.g*., increased sensitivity in amplification and detection assays and allowing for a larger proportion of an extracted sample containing residual detergent(s) to be used in an amplification reaction mixture. In other aspects, the present invention helps to stabilize a lyophilized polymerase formulation due to α-cyclodextrin acting as a secondary lyoprotectant (*e.g.,* in situations wherein increasing the concentration of a primary lyoprotectant leads to incomplete reconstitution of the lyophilized product).

In certain embodiments, the polymerase formulation is an aqueous formulation. Such formulations may be, for example, a pre-lyophilized formulation or one that has been reconstituted from a lyophilized form. In some variations, the formulation is provided as an aqueous solution comprising at least one polymerase, α-cyclodextrin at a concentration of from about 10 mg/mL to about 40 mg/mL, polysorbate 20 at a concentration of from about 0.002% to about 0.05% (v/v), and at least one buffering agent. In some embodiments, α-cyclodextrin is present at a concentration of from about 10 mg/mL to about 35 mg/mL, from about 10 mg/mL to about 30 mg/mL, from about 10 mg/mL to about 25 mg/mL, from about 10 mg/mL to about 20 mg/mL, from about 10 mg/mL to about 15 mg/mL, from about 12 mg/mL to about 40 mg/mL, from about 12 mg/mL to about 35 mg/mL, from about 12 mg/mL to about 30 mg/mL, from about 12 mg/mL to about 25 mg/mL, from about 12 mg/mL to about 20 mg/mL, from about 12 mg/mL to about 15 mg/mL, from about 16 mg/mL to about 40 mg/mL, from about 16 mg/mL to about 35 mg/mL, from about 16 mg/mL to about 30 mg/mL, from about 16 mg/mL to about 25 mg/mL, or from about 15 mg/mL to about 20 mg/mL; in more specific variations, α-cyclodextrin is present at a concentration of about 20 mg/mL, about 17.5 mg/mL, or about 12.5 mg/mL. In some embodiments, polysorbate 20 is present at a concentration of from about 0.002% to about 0.05% (v/v), from about 0.002% to about 0.04% (v/v), from about 0.002% to about 0.03% (v/v), from about 0.003% to about 0.05% (v/v), from about 0.003% to about 0.04% (v/v), or from about 0.003% to about 0.03% (v/v); in more specific variations, polysorbate 20 is present at a concentration of about 0.0042% (v/v), about 0.0035% (v/v), about 0.0026% (v/v), or about 0.02% (v/v).

Suitable polymerases for use in accordance with the present invention include DNA-dependent DNA polymerases, RNA-dependent DNA polymerases (reverse transcriptases), RNA polymerases, and enzymes having more than one type of polymerase activity, and the enzyme can be thermolabile or thermostable. Mixtures of two or more enzymes can also be used. Exemplary polymerases include DNA-dependent DNA polymerases such as, *e.g.,* DNA Polymerase I ("Pol I"), the Klenow fragment of Pol I, T4, T7, Sequenase^{®} T7, Sequenase^{®} Version 2.0 T7, Tub, Taq, Tth, Pfx, Pfu, Tsp, Tfl, Tli and *Pyrococcus* sp. GB-D DNA polymerases; RNA polymerases such as *E*. *coli,* SP6, T3 and T7 RNA polymerases; and reverse transcriptases such as Avian Myeloblastosis Virus (AMV), Moloney Murine Leukemia Virus (MMLV), RNAse H⁻ MMLV (SuperScript^{®}), SuperScript^{®} II, ThermoScript^{®}, HIV-1, and RAV2 reverse transcriptases. In some embodiments, the formulation includes at least two DNA polymerases such as, for example, a reverse transcriptase (*e.g.,* an MMLV mutant reverse transcriptase) and a DNA-dependent DNA polymerase (*e.g.*, a Taq DNA polymerase).

A buffering agent is typically present at a concentration sufficient to maintain a pH suitable for use of the polymerase in an amplification assay. In some embodiments, a buffering agent is present at a concentration sufficient to maintain a pH in the range of from about 6.0 to about 9.0, from about 6.5 to about 8.5, from about 6.5 to about 8.0, or from about 6.5 to about 7.5. Suitable buffering agents include Tris (2-amino-2-(hydroxymethyl)-1,3-propanediol), PIPES (piperazine-N,N'-bis(2-ethanesulfonic acid)), HEPES (4-(2-hydroxyethyl)-1-piperazineethanesulfonic acid), phosphate, citrate, succinate, and histidine. In certain embodiments, a Tris buffering agent is present at a concentration of about 10 mM to about 100 mM or about 20 mM to about 100 mM. Other suitable concentrations of buffers for formulations in accordance with the present invention can be readily determined by one of ordinary skill in the art.

A polymerase formulation as above may further include one or more additional components. For example, a formulation may further contain nucleotide triphosphates suitable for performing nucleic acid amplification (*e.g.*, dATP, dCTP, dGTP, and dTTP; and/or ATP, CTP, GTP and UTP) and/or a chelating agent. Suitable chelating agents inlcude ethylenediaminetetraacetic acid (EDTA) and ethylene glycol-bis(β-aminoethyl ether)-N,N,N',N'-tetraacetic acid (EGTA). In some embodiments comprising EDTA as the chelating agent, EDTA is present at a concentration of from about 0.05 mM to about 0.5 mM (*e.g.,* at a concentration of about 0.16 mM or about 0.14 mM).

In certain variations, formulations - such as those suitable for lyophilization, reconstituted from lyophilized form, or a lyophilized formulation for reconstitution into an aqueous formulation as described herein - may contain a lyoprotectant. Exemplary lyoprotectants include non-reducing sugars such as, *e.g.,* sucrose, raffinose, or trehalose; glycerol; and amino acids such as, *e.g.,* glycine, arginine, or methionine. The use of lyoprotectants, including selection of appropriate concentrations to prevent unacceptable amounts of degradation and/or aggregation of a carrier molecule upon lyophilization, is generally well-known in the art. A particularly suitable lyoprotectant is trehalose, which may be present in an aqueous formulation, for example, at a concentration of from about 0.2 M to about 0.35 M or from about 0.2 M to about 0.4 M. In some variations comprising trehalose as a lyoprotectant, the trehalose is present at a concentration of about 0.26 M or about 0.3 M.

In some embodiments, a polymerase formulation as described herein is a concentrated preparation of one or more polymerases that may be used as bulk product for preparation of an amplification reaction mixture.

In typical variations, the formulation is stable over extended periods of time. For example, the formulations may be stable for at least about two weeks, at least about one month, at least about two months, at least about three months, or at least about six months. In some embodiments, the formulation is stable for at least about 12 months, at least about 14 months, at least about 18 months, at least about 24 months, or at least about 30 months.

A polymerase formulation as described herein may be stored at temperatures from about -80°C to about 40°C, from about -20°C to about 25°C, from about 0°C to about 25°C, from about 0°C to about 15°C, from about 0°C to about 10°C, or from about 2°C to about 8°C. In various embodiments, the formulation may be stored at about 0°C, 1°C, 2°C, 3°C, 4°C, 5°C, 6°C, 7°C, 8°C, 9°C, or 10°C. Generally, the formulation is stable and retains activity at these ranges. In some variations, the formulation is stable from about -80°C to about 25°C or from about 4°C to about 25°C. In more particular variations, a liquid formulation is stable at a temperature from about -80°C to about -20°C, from about -80°C to about 4°C, or from about -80°C to about 25°C. In other particular variations, a lyophilized formulation is stable at a temperature from about 4°C to about 25°C or from about 4°C to about 40°C. Ranges intermediate to the above recited temperatures, for example, from about 2°C to about 18°C, are also intended to be part of this invention. For example, ranges of values using a combination of any of the above recited values as upper and/or lower limits are intended to be included.

In particular embodiments, for long-term storage, an aqueous formulation as described herein may be aliquoted into, *e.g*., vials, ampules, or other containers and lyophilized according to procedures known in the art. The lyophilized product typically appears as a powder or cake. The containers are then sealed; in some such variations, the seal permits later injection of diluent through the seal and into the container. Methods of preparing such lyophilized polymerase formulations from the aqueous formulation, as well as the lyophilized formulations prepared by such methods, are additional aspects of the present invention. In yet another aspect, the present invention provides a stabilized, lyophilized polymerase formulation that enables reconstitution into an aqueous far-red dye probe formulation as described herein.

Methods of preparing an aqueous polymerase formulation from a lyophilized formulation are described. Such methods generally include (a) providing a lyophilized polymerase formulation as described herein and, (b) dissolving the lyophilized polymerase formulation in a suitable diluent to provide a reconstituted formulation. Suitable diluents may be readily selected by a skilled artisan and may include, for example, water or an aqueous solution containing a buffering agent (*e.g.,* Tris).

In certain aspects of the present invention, a first sealed container containing a lyophilized polymerase formulation that enables reconstitution into an aqueous polymerase formulation comprising: at least one polymerase; α-cyclodextrin at a concentration of from about 10 mg/mL to about 40 mg/mL; polysorbate 20 at a concentration of from about 0.002% to about 0.05% (v/v); at least one buffering agent; and a lyoprotectant is provided in a kit with one or more other components such as, for example, a second container containing a diluent. A polymerase formulation may be packaged in a variety of different embodiments, and those skilled in the art will appreciate that the invention embraces many different kit configurations. For example, a kit may further include a container containing one or more amplification oligomers for amplifying the target region, optionally with one or more detection probes for detecting the target region to be amplified. A kit may contain other reagents suitable for performing *in vitro* amplification such as, *e.g.,* buffers, salt solutions, and/or appropriate nucleotide triphosphates (*e.g*., dATP, dCTP, dGTP, dTTP, ATP, CTP, GTP, UTP). In certain embodiments, the kit further includes a set of instructions for practicing methods in accordance with the present invention, where the instructions may be associated with a package insert and/or the packaging of the kit or the components thereof.

In other aspects, the present disclosure provides an amplification reaction mixture. Such reaction mixtures may, for example, be prepared using an aqueous formulation as described herein as a concentrated bulk product. A reaction mixture of the present disclosure typically comprises at least one polymerase, α-cyclodextrin at a concentration of from about 5 mg/mL to about 20 mg/mL, polysorbate 20 at a concentration of from about 0.001%to about 0.025% (v/v), and at least one buffering agent. α-cyclodextrin may be present at a concentration of from about 5 mg/mL to about 17.5 mg/mL, from about 5 mg/mL to about 15 mg/mL, from about 5 mg/mL to about 12.5 mg/mL, from about 8 mg/mL to about 20 mg/mL, from about 8 mg/mL to about 17.5 mg/mL, from about 8 mg/mL to about 15 mg/mL, or from about 8 mg/mL to about 12.5 mg/mL; in more specific variations, α-cyclodextrin may be present at a concentration of about 10 mg/mL. Polysorbate 20 may be present at a concentration of from about 0.001% to about 0.02% (v/v), from about 0.001% to about 0.015% (v/v), from about 0.0015% to about 0.025% (v/v), from about 0.0015% to about 0.02% (v/v), or from about 0.0015% to about 0.015% (v/v); in mores specific variations, polysorbate 20 is present at a concentration of about 0.002% (v/v), about 0.0021% (v/v), or about 0.01% (v/v).

Suitable polymerases for use in a reaction mixture include those suitable for a polymerase formulation as discussed above. For example, the reaction mixture may include at least two DNA polymerases such as, for example, a reverse transcriptase (*e.g*., an MMLV mutant reverse transcriptase) and a DNA-dependent DNA polymerase (*e.g*., a Taq DNA polymerase).

A buffering agent is typically present in a reaction mixture at a concentration sufficient to maintain a pH suitable for use of the polymerase in an amplification assay. A buffering agent may be present at a concentration sufficient to maintain a pH in the range of from about 6.0 to about 9.0, from about 6.5 to about 8.5, from about 6.5 to about 8.0, or from about 6.5 to about 7.5. Suitable buffering agents include buffering agents suitable for a polymerase formulation as discussed above. A Tris buffering agent may be present at a concentration of about 5 mM to about 50 mM or about 10 mM to about 50 mM. Other suitable concentrations of buffers for reaction mixtures in accordance with the present invention can be readily determined by one of ordinary skill in the art.

An amplification reaction mixture may contain a lyoprotectant (such as, for example, where an amplification reaction mixture is prepared using an aqueous polymerase formulation that has been reconstituted from a lyophilized form). The lyoprotectant may be trehalose, which may be present in a reaction mixture, for example, at a concentration of from about 0.1 M to about 0.2 M. In some variations of a reaction mixture comprising trehalose, the trehalose may be present at a concentration of about 0.15 M.

A reaction mixture as above may further include one or more additional components for performing an amplification assay. For example, a reaction mixture may further contain at least one amplification oligomer configured to amplify a target region of a target nucleic acid. The reaction mixture may contain at least two amplification oligomers (*e.g.*, a first amplification oligomer and a second amplification oligomer) configured to amplify the target region in multiple cycles of the amplification assay. A reaction mixture may contain at least one detection probe oligomer configured to specifically hybridize to a target sequence contained within a target region to be amplified. A detection probe oligomer for use in an amplification reaction mixture typically comprises a detectable label such as, for example, a chemiluminescent compound or fluorophore. At least two amplification oligomers and at least one detection probe oligomer in the reaction mixture may be configured to amplify a target region and detect the amplification product in real time such as, for example, in a real-time PCR assay or a real-time transcriptional-mediated amplification (TMA) assay. Particularly suitable detection probe oligomers for real-time detection include oligomers comprising a fluorescent label and a non-fluorescent quencher (*e.g*., a hydrolysis probe, molecular torch, or molecular beacon).

A reaction mixture may include one or more additional, suitable amplification assay components selected from nucleotide triphosphates (*e.g*., dATP, dCTP, dGTP, and dTTP; and/or ATP, CTP, GTP and UTP), a chelating agent (*e.g.,* EDTA or EGTA), an inorganic salt (*e.g.,* KCl), and a cofactor. In some variations, the chelating agent may be EDTA, which may be present, for example, at a concentration of from about 0.025 mM to about 0.25 mM (*e.g.,* at a concentration of about 0.08 mM). A particularly suitable cofactor is magnesium chloride, which may be present, for example, at a concentration of from about 1 mM to about 5 mM.

A reaction mixture as above may further include a target nucleic acid or a sample suspected of containing a target nucleic acid. For example, a reaction mixture as above comprising at least one amplification oligomer may further contain the target nucleic acid targeted by the at least one amplification oligomer, or a sample suspected of containing the target nucleic acid. The target nucleic acid can be any target nucleic acid of interest and may be RNA or DNA. The target nucleic acid may be from a pathogen such as a virus, bacterium, protozoan, fungus, or other microorganism responsible for disease in humans or animals.

A reaction mixture as above may further include at least one detergent. For example, a reaction mixture may contain at least one detergent that is introduced into the reaction mixture from a sample containing or suspected of containing the target nucleic acid, wherein the sample contains one or more residual detergents used in sample extraction. In certain variations, the detergent may be an anionic detergent such as, for example, a dodecyl sulfate salt (*e.g*., sodium dodecyl sulfate, potassium dodecyl sulfate, or lithium dodecyl sulfate (also referred to herein as lithium lauryl sulfate)) or N-lauroyl sarcosine. A sample containing at least one detergent may constitute at least about 20% or at least about 40% of the reaction mixture by volume. In more specific variations, a sample containing at least one detergent may constitute from about 40% to about 50%, from about 50% to about 70%, from about 20% to about 33%, about 42.9%, or about 62.5% of the reaction mixture by volume.

In other aspects, the present disclosure provides a method of preparing an amplification reaction mixture. A method of preparing the reaction mixture may include (a) providing an aqueous polymerase formulation as described above, and (b) mixing the aqueous polymerase formulation with a second formulation containing at least one amplification oligomer (*e.g*., at least two amplification oligomers) configured to amplify a target region of a target nucleic acid. A method of preparing the reaction mixture may include (a) providing a lyophilized polymerase formulation as described above, (b) dissolving the lyophilized polymerase formulation in a diluent to provide a reconstituted formulation; and (c) mixing the reconstituted formulation with a second formulation comprising at least one amplification oligomer (*e.g.*, at least two amplification oligomers) configured to amplify a target region of a target nucleic acid. Suitable polymerases for include those suitable for a polymerase formulation or reaction mixture as discussed above. In certain variations, a method as above may further include mixing the aqueous or reconstituted formulation with one or more additional amplification assay components. Such additional component(s) may be contained within the second formulation or may be contained in one or more separate formulations mixed with the aqueous or reconstituted polymerase formulation. Such one or more additional components may include (i) at least one detection probe oligomer configured to specifically hybridize to a target sequenced contained within the target region (*e.g.,* a probe comprising a label such a chemiluminescent or fluorescent label, or comprising a fluorescent label and a non-fluorescent quencher), (ii) nucleotide triphosphates (*e.g.*, dATP, dCTP, dGTP, and dTTP; and/or ATP, CTP, GTP and UTP), (iii) at least one chelating agent (*e.g.,* EDTA or EGTA), and/or (iv) at least one cofactor (*e.g.,* magnesium chloride). In some embodiments, the aqueous or reconstituted polymerase formulation is a formulation further comprising nucleotide triphosphates and/or a chelating agent as described herein, and the one or more additional components mixed with the aqueous or reconstituted formulation includes (i) at least one detection probe oligomer configured to specifically hybridize to a target sequenced contained within the target region, and/or (ii) at least one cofactor.

A method for preparing a reaction mixture may further include mixing the aqueous or reconstituted formulation, or a mixture as above comprising the aqueous or reconstituted formulation, with a sample containing or suspected of containing a target nucleic acid. The sample may be an extracted sample derived from processing a biological sample with one or more detergents, whereby the extracted sample contains one or more detergents as residual components. The residual detergent may be an anionic detergent such as, for example, a dodecyl sulfate salt (*e.g*., sodium dodecyl sulfate, potassium dodecyl sulfate, or lithium dodecyl sulfate) or N-lauroyl sarcosine. An extracted sample containing at least one detergent may constitute at least about 20% or at least about 40% of the reaction mixture by volume (*e.g*., from about 40% to about 50%, from about 50% to about 70%, from about 20% to about 33%, about 42.9%, or about 62.5% of the reaction mixture by volume).

Final assay concentrations of the mixture components may include concentrations as previously described herein for an amplification reaction mixture. For example, α-cyclodextrin may be mixed to a concentration of from about 5 mg/mL to about 20 mg/mL; polysorbate 20 may be mixed to a concentration of from about 0.001% to about 0.025% (v/v); Tris, if present, may be mixed to a concentration of from about 5 mM to about 50 mM; EDTA, if present, may be mixed to a concentration of from about 0.025 mM to about 0.25 mM; trehalose, if present, may be mixed to a concentration of from about 0.1 M to about 0.2 M; and/or magnesium chloride, if present, may be mixed to a concentration of from about 1 mM to about 5 mM.

In other aspects, the present disclosure provides a method for performing an amplification reaction. The method may include (a) providing a sample containing or suspected of containing a target nucleic acid; (b) contacting the sample with an aqueous mixture comprising at least one polymerase, α-cyclodextrin, polysorbate 20, at least one buffering agent (*e.g*., Tris), nucleotide triphosphates suitable for performing nucleic acid amplification (*e.g.*, dATP, dCTP, dGTP, and dTTP; and/or ATP, CTP, GTP and UTP), at least one cofactor (*e.g.*, magnesium chloride), and at least one amplification oligomer (*e.g.*, at least two amplification oligomers) configured to amplify a target region of the target nucleic acid, wherein contacting the sample with the aqueous mixture provides a reaction mixture in which the α-cyclodextrin is present at a concentration of from about 5 mg/mL to about 20 mg/mL and the polysorbate 20 is present at a concentration of from about 0.001% to about 0.025% (v/v); and (c) using the reaction mixture to perform an *in vitro* nucleic acid amplification reaction, wherein the target nucleic acid, if present in the sample, is used as a template for generating one or more amplicons corresponding to the target region. Suitable polymerases include those suitable for a polymerase formulation or reaction mixture as discussed above. In certain variations, a method as above may further include contacting the sample with one or more additional assay components such as, *e.g.,* at least one detection probe oligomer configured to specifically hybridize to a target sequence contained within the one or more amplicons, and/or (iii) at least one chelating agent (*e.g.*, EDTA or EGTA). The aqueous mixture comprising the polymerase may further contain a lyoprotectant (*e.g.*, used in lyophilized formulation from which an aqueous formulation is reconstituted) such as, for example, trehalose. Final amplification assay concentrations of the mixture components may include concentrations as previously described herein for an amplification reaction mixture. Amplifying the target region of the target nucleic acid can be accomplished using a variety of known nucleic acid amplification reactions, including, for example, transcription-associated amplification *(e.g.,* transcription-mediated amplification (TMA) or nucleic acid sequence-based amplification (NASBA)), Polymerase Chain Reaction (PCR), replicase-mediated amplification, and Ligase Chain Reaction (LCR), to name a few.

In the method for performing an amplification reaction as above, the sample may be an extracted sample derived from processing a biological sample with one or more detergents, whereby the extracted sample contains one or more detergents as residual components. The residual detergent may be an anionic detergent such as, for example, a dodecyl sulfate salt (*e.g.,* sodium dodecyl sulfate, potassium dodecyl sulfate, or lithium dodecyl sulfate) or N-lauroyl sarcosine. The extracted sample containing at least one detergent may constitute at least about 20% or at least about 40% of the reaction mixture by volume (*e.g.,* from about 40% to about 50%, from about 50% to about 70%, from about 20% to about 33%, about 42.9%, or about 62.5% of the reaction mixture by volume).

A method for performing an amplification reaction as above may further include purifying the target nucleic acid from other components in the sample before amplification. Such purification may include methods of separating and/or concentrating organisms contained in a sample from other sample components, or removing or degrading non-nucleic acid sample components, *e.g*., protein, carbohydrate, salt, lipid, *etc.* A target nucleic acid may be captured specifically or non-specifically and separated from other sample components. Target capture typically occurs in a solution phase mixture that contains one or more capture probe oligomers that hybridize to a target sequence of the target nucleic acid under hybridizing conditions. For embodiments comprising a capture probe tail, a target capture-probe complex may be captured by using hybridization conditions under which the capture probe tail hybridizes to an immobilized probe. Certain embodiments may use a particulate solid support, such as paramagnetic beads. Selective and non-specific target capture methods are also described, *e.g*., in US Patent No. 6,110,678 and International Patent Application Pub. No. WO 2008/016988. A purification step (*e.g.,* a purification step comprising specific or non-specific target capture) may utilize one or more detergents such as, for example, an anionic detergent (*e.g*., sodium dodecyl sulfate or lithium lauryl sulfate).

A method for amplifying a target nucleic acid as above may further include detecting the one or more amplicons. A detection step may be performed using any of a variety of known techniques to detect a signal specifically associated with the amplified target sequence, such as, *e.g.,* by hybridizing the amplification product with a labeled detection probe and detecting a signal resulting from the labeled probe (including from label released from the probe following hybridization in some embodiments). The labeled probe comprises a second moiety, such as a quencher or other moiety that interacts with the first label. Detection may be performed after the amplification reaction is completed or may be performed simultaneously with amplifying the target region, *e.g.,* in real time. In certain variations that use real-time detection, the detection probe may be a hairpin probe such as, for example, a molecular beacon, molecular torch, or hybridization switch probe that is labeled with a reporter moiety that is detected when the probe binds to amplified product (*e.g.,* a dual-labeled hairpin probe comprising both a fluorescent label and a quenching moiety). For real-time detection, the detection probe may be a linear oligomer such as, *e.g.,* an oligomer labeled with both a fluorophore and a quenching moiety (*e.g.,* a TaqMan probe). Such probes may comprise target-hybridizing sequences and non-target-hybridizing sequences. Various forms of such probes have been described previously (*see, e.g.,* US Patent Nos. 5,210,015; 5,487,972; 5,118,801; 5,312,728; 5,925,517; 6,150,097; 6,849,412; 6,835,542; 6,534,274; and 6,361,945; and US Patent Application Pub. Nos. 20060068417A1 and 20060194240A1).

### Examples

The following examples are provided to illustrate certain disclosed embodiments and are not to be construed as limiting the scope of this disclosure in any way.

### Example 1

Several PCR reaction mixtures were prepared for testing with samples containing a detergent. An initial master mix was prepared to contain DNA polymerase at 0.46 U/µL, Reverse Transcriptase at 0.5 U/µL, RNase inhibitor at 0.2 U/µL, dNTPs at 0.25 mM each, dUTP at 0.05 mM, inorganic salts including KCl and MgCl₂ at 81 and 5.1 mM, EDTA at 0.1 mM., and several primers and probes (SEQ ID NOs:8 to 14 at 0.64 µM to 1.29 µM) for amplification and detection of influenza A target nucleic acids. A second master mix was prepared to contain DNA polymerase at 1.0 U/µL, Reverse Transcriptase at 3.2 U/µL, dNTPs at 0.53 mM, dUTP at 1.06 mM, inorganic salts including KCl and MgCl₂ at 173 and 10.93 mM, EDTA at 0.21 mM, and several primers and probes (SEQ ID NOs:8 to 14 at 0.64 µM to 1.29 µM) for amplification and detection of the same target nucleic acid. The initial master mix was separated into conditions (A) through (F), wherein conditions (B) and (D) further contained polysorbate 20 at 0.025% (v/v), conditions (B), (C) and (F) further contained 12.5 mg/mL α-cyclodextrin, condition (C) further contained polysorbate 20 at 0.13% (v/v), and condition (E) further contained 50 mg/mL α-cyclodextrin.

Samples were prepared by spiking 8.3 copies/mL of an influenza A *in vitro transcript* target nucleic acid (SEQ ID NO:7) into a suitable media, *e.g.,* Micro Test M4 media (Remel Inc. Cat. No. R12500), Micro Test M5 Viral Transport Medium (Remel, Inc. Cat. No. R12515), Micro Test M6 Viral Transport Medium (Remel, Inc. Cat. No. R12530), Micro Test M4RT Viral Transport Medium (Remel, Inc. Cat. No. R12505), or Copan Universal Transport Medium (Copan Diagnostics, Inc., Cat. No. 330C). The target nucleic acid was an RNA transcript of SEQ ID NO:7. 360 µL of the prepared sample was separately incubated in a final reaction volume of 936 µL in a buffered reagent containing 100 µg of poly-T (SEQ ID NO:2) coated magnetic microparticles and 20 picomoles of target capture oligomer (SEQ ID NO:1) to bind the target nucleic acids to a magnetic solid support. The magnetic microparticles and bound nucleic acid were separated out of the solution by the application of a magnetic field, allowing the supernatant to be removed from the [captured target]:[capture probe]:[magnetic microparticle] combination. The magnetic microparticles were then resuspended in wash buffer. The resuspended microparticles were subjected to one more round of separation, supernatant removal, and resuspension in wash buffer. After separation and removal of the second round of wash buffer, the microparticles were incubated in 50 uL of elution buffer (5 mM Tris in water with preservatives). The magnetic particles were separated by the application of a magnetic field and an eluate containing nucleic acid was recovered. The eluate was divided into separate containers and one of the eluate portions was further spiked with 30% (v/v) of wash buffer to produce eluates containing 100 mg/mL of sodium dodecyl sulfate to simulate carry over of wash buffer from the upstream sample processing step. Detergents that are present in wash buffers are known to inhibit or diminish nucleic acid amplification reactions.

Each of the above master mix conditions (A) to (F) were added to a well of a multi-well plate and combined with an aliquot of the wash-buffer-spiked eluate and with an eluate without wash buffer (20 µL master mix, 10 µL eluate, 30 µL total reaction volume). Each condition was prepared in replicates of 20, except for condition (E), which was prepared in replicate of 19. Real-time amplification and detection reactions were set up for the 20 (19) replicates per condition and the reactions were performed using a thermal cycling instrument (Panther Fusion Instrument, Hologic, Inc., San Diego, CA). Results are presented in **Table 1,** below.

**Table 1**

| **Master Mix Condition** | **Wash Buffer Spike** | **N Positives** | **Average Ct** | **SD Ct** | **Average RFU** | **SD RFU** |
|---|---|---|---|---|---|---|
| (A) | No | 20/20 | 33.2 | 0.8 | 22225 | 3134 |
| (A) | Yes | 1/20 | 38.2* | n/a | 791 | n/a |
| (B) | No | 20/20 | 32.2 | 0.5 | 25043 | 2460 |
| (B) | Yes | 20/20 | 32.5 | 0.9 | 24276 | 2879 |
| (C) | No | 20/20 | 32.4 | 0.5 | 22664 | 3118 |
| (C) | Yes | 20/20 | 32.3 | 0.5 | 23200 | 2561 |
| (D) | Yes | 20/20 | 34.2 | 0.6 | 15690 | 1785 |
| (E) | Yes | 19/19 | 34.2 | 0.8 | 19965 | 2826 |
| (F) | Yes | 20/20 | 35.2 | 1.2 | 19346 | 3674 |

| | | | | | | |
|---|---|---|---|---|---|---|
| ***Ct value is for a single replicate, not an average** | | | | | | |

These data show that the addition of α-cyclodextrin and polysorbate 20 into the reaction mixture mitigated the negative/inhibitory effect that wash buffer detergents have on the nucleic acid amplification reactions. These data also show that the combination of these two reagents had a synergistic mitigating effect on the wash-buffer-induced inhibition relative to the mitigating effects observed using either α-cyclodextrin alone or polysorbate 20 alone.

### Example 2

A PCR master mixture was prepared substantially as described in Example 1 and referred to as a second master mixture. In this master mixture the primers and probe were SEQ ID NOs:4 to 6 and the master mixture further contained 0.4 M trehalose. The master mixture was separated into separate conditions, wherein condition (G1) further contained 26.7 mg/mL α-cyclodextrin and 0.0057% (v/v) polysorbate 20, condition (G2) further contained 28.0 mg/mL α-cyclodextrin and 0.0063% (v/v) polysorbate 20, condition (G3) further contained 25.4 mg/mL α-cyclodextrin and 0.0051% (v/v) polysorbate 20, and (G4) contained no further additives.

Seasonal coronavirus type OC43 (Zeptometrix Cat No 0810024CF) was spiked into a whole blood sample matrix or into a sample transport media (STM) to provide a final viral titer of 0.125 TCID₅₀/mL in each sample type. 400 uL of the whole blood matrix or the STM matrix were separately incubated in a final reaction volume of 976 µL in a buffered reagent containing 100 µg of poly-T (SEQ ID NO:2) coated magnetic microparticles and 20 picomoles of target capture oligomer (SEQ ID NO:3) to bind the target nucleic acids to a magnetic solid support. The magnetic microparticles and bound nucleic acid were separated as generally described in Example 1, and an eluate containing target nucleic acid was recovered. Eluates were then divided into two equal parts, and to one part the eluate was further spiked with 30% (v/v) of wash buffer to produce eluates containing 100 mg/mL of sodium dodecyl sulfate to simulate carry over of wash buffer from the upstream sample processing step. Detergents that are present in wash buffers are known to inhibit or diminish nucleic acid amplification reactions.

Each of the above master mix conditions (G1) to (G4) were added to a well of a multi-well plate and combined with an aliquot of the wash buffer spiked eluates or an aliquot of the eluates that did not receive a wash buffer spike (15 µL master mix, 25 µL eluate, 40 µL total reaction volume, except the (G4) condition was 20 µL master mix, 5 µl eluate, 25 µL total volume). Each condition was prepared in replicates of 24, except that condition (G1) with the unspiked eluate was prepared in a replicate of 20. RT-PCT reactions were performed using a thermal cycling instrument (Panther Fusion Instrument, Hologic, Inc., San Diego, CA). Condition (G4) is prepared to have 5.3 times less target nucleic acid than it would if prepared using 15 µL master mix and 25 µL eluate. Thus, under identical reaction conditions, (G4) has a Ct that is higher than it would if prepared using the alternative reaction condition (theoretically a Ct of 2.4). Results are presented in **Table 2,** below.

**Table 2**

| **Condition** | **Sample Type** | **Spike** | **Percent Positive** | **Avg Ct** | **Avg RFU** |
|---|---|---|---|---|---|
| (G1)a | Whole Blood | 100 mg/mL SDS | 100% | 32.4 | 32,676 |
| (G1)b | Whole Blood | none | 100% | 33.1 | 23,968 |
| (G1)c | STM | 100 mg/mL SDS | 100% | 30.8 | 35984 |
| (G1)d | STM | none | 100% | 31.5 | 33649 |
| (G2)a | Whole Blood | 100 mg/mL SDS | 100% | 32.2 | 34,703 |
| (G2)b | Whole Blood | none | 100% | 33.1 | 25,639 |
| (G2)c | STM | 100 mg/mL SDS | 95.8% | 30.7 | 28236 |
| (G2)d | STM | none | 100% | 30.6 | 30858 |
| (G3)a | Whole Blood | 100 mg/mL SDS | 100% | 32.8 | 31,078 |
| (G3)b | Whole Blood | none | 100% | 33.3 | 23,144 |
| (G3)c | STM | 100 mg/mL SDS | 100% | 29.9 | 34972 |
| (G3)d | STM | none | 95.8% | 31.5 | 31400 |
| (G4)a | Whole Blood | 100 mg/mL SDS | 91.7% | 37.5 | 15,386 |
| (G4)b | Whole Blood | none | 100% | 36.3 | 14,841 |
| (G4)c | STM | 100 mg/mL SDS | 100% | 36.3 | 20892 |
| (G4)d | STM | none | 100% | 34.7 | 21400 |

These data show lower Ct values and higher RFU values for conditions (G1), (G2), and (G3) compared to condition (G4). Comparing against condition (G4)a, these data show Ct improvement of -5.1, -5.3, and -4.7 and show RFU improvement of +112%, +127%, and +102% for conditions (G1)a, (G2)a, and (G3)a, respectively. Comparing against (G4)b these data show Ct improvement of -3.2, - 3.4, and -3.0 and show RFU improvement of +61%, +72%, and +56% for conditions (G1)b, (G2)b, and (G3)b, respectively. Comparing against (G4)c these data show Ct improvement of -5.5, -5.5, and -6.4 and show RFU improvement of +72%, +35%, and +67% for conditions (G1)c, (G2)c, and (G3)c, respectively. Comparing against (G4)d these data show Ct improvement of -3.2, -4.1, and -3.2 and show RFU improvement of +57%, +44%, and +46% for conditions (G1)d, (G2)d, and (G3)d, respectively. These results show substantial improvement in Ct when comparing samples containing an inhibitory substance and treated with a range of concentrations of α-cyclodextrin and of polysorbate 20 to samples containing an inhibitory substance without such treatment.

### Example 3

Seasonal coronavirus type OC43 (Zeptometrix Cat No 0810024CF) was spiked into several plasma and serum samples to provide a final OC43 viral titer of 70 TCID₅₀/mL in a total sample volume of 1.15 mL. Spiked samples were then combined with a reagent comprising 10% (v/v) lithium lauryl sulfate. Other components of this reagent included buffer, salts, SEQ ID NO:2-coated magnetic particles, SEQ ID NO:1, and a target capture oligomer (SEQ ID NO:3). Each sample was mixed on an orbital shaker (approx. 200 rpm, 1 min). The samples were then incubated in a water bath at 43 °C for 4 min, then at 63 °C for 29 min, and then at 43 °C for 15 min. The magnetic particles were separated by the application of a magnetic field and an eluate containing nucleic acid was recovered. Two reverse transcription, PCR reaction mixtures were prepared, each containing SEQ ID NOs:4, 5, & 6, a DNA polymerase, a reverse transcriptase dNTPs, inorganic salts, BSA, and RNase inhibitor. One of the RT-PCR reaction mixtures further contained 12.5 mg/mL polysorbate 20, and 0.0026% (v/v) α-cyclodextrin. 20 µL of an RT-PCR reaction mixture was combined with 5 µL of eluate from each sample type to prepare the following four conditions: (A) plasma sample eluate with an RT-PCR reaction mixture containing polysorbate 20 and α-cyclodextrin, (B) plasma sample eluate with an RT-PCR reaction mixture - neat, (C) serum sample eluate with an RT-PCR reaction mixture containing polysorbate 20 and α-cyclodextrin, and (D) serum sample eluate with an RT-PCR reaction mixture - neat. RT-PCR reactions were set up in replicates of 9 to 11 (see **Table 3)** and spiked with an internal control. RT-PCR reactions were performed, and the data is presented in **Table 3.**

**Table 3**

| **Condition** | **No. Target Positive** | **Average Ct (SD Ct)** | **Average RFU (SD RFU)** | **No. IC Positive** |
|---|---|---|---|---|
| A | 9/9 | 22.9 (0.19) | 24,160.33 (694.11) | 9/9 |
| B | 11/11 | 27.51 (1.90) | 21,580.55 (638.38) | 11/11 |
| C | 10/10 | 27.39 (0.19) | 23,062.60 (418.26) | 10/10 |
| D | 11/11 | 34.69 (2.39) | 19,611.64 (2,025.17) | 8/11 |

The data in **Table 3** show that in the presence of residual LLS, the samples generated RT-PCR results with higher Ct, lower RFU and more sample-to-sample variability than what was generated for the reactions containing reagents that mitigate the effects of LLS. This indicates that addition of α-cyclodextrin and polysorbate 20 significantly helped in the detection of RNA by more than 1 log (more than 4 Ct lower with additives).

### Example 4

A series of aqueous mixtures containing α-cyclodextrin and polysorbate 20 were prepared. This series of mixtures were set-up with polysorbate 20 at a constant concentration and combined with several concentrations of α-cyclodextrin, or with α-cyclodextrin at a constant concentration and combined with several different concentrations of polysorbate 20 **(Table 4).** Each combination in mixtures 1 to 7 was effective at mitigating the inhibitory effect of a detergent in a nucleic acid amplification reaction mixture. Each aqueous mixture was held on ice for 1 hour and then the mixtures were centrifuged to evaluate whether a precipitant was present. Varying levels of precipitates were seen in mixtures 1-6 following the 1-hour incubation on ice.

**Table 4**

| | Mix 1 | Mix 2 | Mix 3 | Mix 4 | Mix 5 | Mix 6 | Mix 7 |
|---|---|---|---|---|---|---|---|
| Polysorbate 20 (% v/v) | 0.02 | 0.02 | 0.02 | 0.02 | 0.015 | 0.01 | 0.005 |
| α-Cyclodextrin (mg/mL) | 20 | 15 | 10 | 5 | 20 | 20 | 20 |

Several dried RT-PCR reaction mixtures were prepared to contain various concentrations of α-cyclodextrin and polysorbate 20. An RT-PCR master mixture was prepared to contain DNA polymerase at 1.0 U/µL, Reverse Transcriptase at 3.2 U/µL, 0.4M trehalose, dNTPs at 0.53 mM, dUTP at 1.06 mM, EDTA at 0.21 mM, 1.6 µM of each of SEQ ID NOs:4 & 5, and 1.07 µM of SEQ ID NO:6. This mastermix was then divided into separate conditions, wherein condition (1) contained 16 mg/mL α-cyclodextrin and 0.0034% (v/v) polysorbate 20, condition (2) contained 20 mg/mL α-cyclodextrin and 0.02% (v/v) polysorbate 20, and condition (3) contained neither α-cyclodextrin nor polysorbate 20. Each of these conditions were then aliquoted at 24 µL into several reaction wells per condition and the aliquots were dried using lyophilization technology to form single unit dose lyophilization pellets.

Seasonal coronavirus type OC43 (Zeptometrix Cat No 0810024CF) was spiked into a whole blood sample matrix, a plasma sample matrix, a serum sample matrix, or into a sample transport media (STM) to provide a final viral titer of 14.2 TCID₅₀/mL in each sample type. 400 µL of the whole blood matrix or 200 µL of the plasma or serum matrices, or 360 µL of the STM matrix were separately incubated in a final reaction volume of 936 µL in a buffered reagent containing 100 µg of poly-T (SEQ ID NO:2) coated magnetic microparticles and 20 picomoles of target capture oligomer (SEQ ID NO:3) to bind the target nucleic acids to a magnetic solid support. The magnetic microparticles and bound nucleic acid were separated as generally described in Example 1, and an eluate containing target nucleic acid was recovered. Eluates were then divided into two equal parts, and to one part the eluate was further spiked with 30% (v/v) of wash buffer to produce eluates containing 100 mg/mL of sodium dodecyl sulfate to simulate carry over of wash buffer from the upstream sample processing step.

Each single unit does lyophilized pellets for conditions (1) to (3) was rehydrated with 25 µL of a HEPES buffered solution containing KCl and MgCl₂, and 15 µL of the rehydrated reaction mixture was combined with 25 µL of an eluate for conditions (1) and (2), while 20 µL of the rehydrated reaction mixture (3) was combined with 5 µL of eluate (all in replicates of 6). RT-PCT reactions were performed using a thermal cycling instrument. Results are presented in **Table 5.**

**Table 5**

| **Condition** | **Sample Type** | **Spike** | **Percent Positive** | **Avg Ct** | **Avg RFU** |
|---|---|---|---|---|---|
| (1)a | Whole Blood | none | 100% | 28.9 | 32,371 |
| (1)b | Whole Blood | 100 mg/mL SDS | 100% | 28.1 | 37,904 |
| (1)c | Plasma | none | 100% | 26.2 | 33,617 |
| (1)d | Plasma | 100 mg/mL SDS | 100% | 25.5 | 40,177 |
| (1)e | Serum | none | 100% | 27.6 | 32,642 |
| (1)f | Serum | 100 mg/mL SDS | 100% | 31.4 | 40,915 |
| (1)g | STM | none | 100% | 26.5 | 33,175 |
| (1)h | STM | 100 mg/mL SDS | 100% | 27.6 | 35,475 |
| (2)a | Whole Blood | none | 100% | 29.0 | 33,747 |
| (2)b | Whole Blood | 100 mg/mL SDS | 100% | 28.3 | 44,044 |
| (2)c | Plasma | none | 100% | 25.1 | 36,269 |
| (2)d | Plasma | 100 mg/mL SDS | 100% | 26.1 | 37,614 |
| (2)e | Serum | none | 100% | 32.3 | 33,538 |
| (2)f | Serum | 100 mg/mL SDS | 100% | 29.5 | 37,754 |
| (2)g | STM | none | 100% | 27.8 | 32,255 |
| (2)h | STM | 100 mg/mL SDS | 100% | 27.4 | 36,338 |
| (3)a | Whole Blood | none | 100% | 31.5 | 22,446 |
| (3)b | Whole Blood | 100 mg/mL SDS | 100% | 32.2 | 25,581 |
| (3)c | Plasma | none | 100% | 28.8 | 22,824 |
| (3)d | Plasma | 100 mg/mL SDS | 100% | 29.6 | 25,468 |
| (3)e | Serum | none | 100% | 35.3 | 21,528 |
| (3)f | Serum | 100 mg/mL SDS | 100% | 34.1 | 24,205 |
| (3)g | STM | none | 100% | 29.8 | 21,130 |
| (3)h | STM | 100 mg/mL SDS | 100% | 32.5 | 24,390 |

These data show lower Ct values and higher RFU values for the reactions containing combinations of α-cyclodextrin and polysorbate 20 compared to comparable reactions without these additives.

### Example 5

An amplification reagent mixture was prepared, consisting of DNA polymerase at 0.65 U/µL, dNTPs at 0.35 mM each, dUTP at 0.7 mM, inorganic salts including KCl and MgCl₂ at 81 and 5.1 mM, trehalose at 0.26 M, Ethylenediaminetetraacetic acid at 0.14 mM, α-cyclodextrin at 17.5 mg/mL and polysorbate 20 at 0.0035%. The reagent mixture either contained primers and probes specific for BK virus (BKV) (SEQ ID NOs:15-17; *see* Table 6) or specific for Epstein-Barr virus (EBV) detection (SEQ ID NOs: 18-20; *see* Table 6).

Samples consisted of human whole blood diluted in blood transport medium in a 1:4 ratio, human urine diluted in urine transport medium in a 1:2 ratio or human plasma undiluted. 400µL of each of these samples were separately incubated in a final reaction volume of 976 µL containing 270 µg of poly-T (SEQ ID NO:2; 5'-(T)14-3') coated magnetic microparticles and 220 picomoles of target capture oligomer (SEQ ID NO:1; 5'-(K)18(T)3(A)30-3') to bind the target nucleic acids to a magnetic solid support. The magnetic microparticles and bound nucleic acid were separated out of the solution by the application of a magnetic field, allowing the supernatant to be removed from the [captured target]:[capture probe]:[magnetic microparticle] combination. The magnetic microparticles were then resuspended in wash buffer. The resuspended microparticles were subjected to one more round of separation, supernatant removal, and resuspension in wash buffer. After separation and removal of the second round of wash buffer, the microparticles were incubated in 50 µL of elution buffer (5 mM Tris in water with preservatives). The magnetic particles were separated by the application of a magnetic field and an eluate containing nucleic acid was recovered.

Each of the above master mix conditions were combined in a well of a multi-well plate (20 µL master mix, 15 µL eluate, 35 µL total reaction volume). Real-time amplification and detection reactions were set up for the extracted samples and the reactions were performed using a thermal cycling instrument (Panther Fusion Instrument, Hologic, Inc., San Diego, CA).

Linearity of the EBV detection was assessed across the range from 2.45 log IU/mL to 9.86 log IU/mL for human whole blood diluted in Blood Transport medium and from 1.98 log IU/mL to 9.26 log IU/mL for undiluted human plasma. Linearity of BKV detection was assessed across the range from 2.11 log IU/mL to 9.38 log IU/mL for human urine diluted in urine transport medium and from 1.80 log IU/mL to 9.08 log IU/mL for undiluted human plasma. Linearity was demonstrated across the range tested for all conditions.

**Table 6. Oligonucleotide Sequences**

| SE Q ID NO | Sequence (5' to 3') | Description |
|---|---|---|
| 1 | KKKKKKKKKKKKKKKKKTTTAAAAAAAAAAAAAAAAAAAAA AA | TCO |
| 2 | TTTTTTTTTTTTTT | IP |
| 3 | RRRRRRRRRRRRRRRTTTAAAAAAAAAAAAAAAAAAAAAAAAA AA | TCO |
| 4 | GATGTGGTGGTTTTGGACAT | OC43 Primer |
| 5 | CTTCTTAGAAAACTTACCCTTAGG | OC43 Primer |
| 6 | AGTTGAAGATGCTTATGACCAGGTGC | OC43 DP |
| | | FAM/BHQ-1 |
| | | 3' blocked |
| 8 | CTTCTAACCGAGGTCGAAACGT | Flu A primer |
| 9 | CCCTTAGTCAGAGGTGACA | Flu A primer |
| 10 | ATAACGTTCCATGGGGCCAA | Flu A primer |
| 11 | CCCATYCTGTTGTATATGAG | Flu A primer |
| 12 | TCAGGCCCCCTCAAAGCCGAGATCGC | Flu A DP |
| | | FAM/BHQ-1 |
| | | 3' blocked |
| 13 | AGCCAUTCCATGAGAGCCTCAAGATC | Flu A DP |
| | | FAM/BHQ-1 |
| | | 3' blocked |
| 14 | CTGGTGCACTTGCCAGTTGUATG | Flu A DP |
| | | FAM/BHQ-1 |
| | | 3' blocked |
| 15 | CCCTACTTGAGCCAARGAACTAA | BKV primer |
| 16 | GGCCTAACWCCTCAAACATATGC | BKV primer |
| 17 | TTAAAGCAGCAAACCCAGCAATAGCC | BKV DP |
| | | CalRed610/BHQ-2 blocked |
| 18 | TTAGGAGACGCCCTCAATCG | EBV primer |
| 19 | TGGCCAGAAATACACCAACA | EBV primer |
| 20 | | EBV DP |
| | CCGTGTATTCCCCCGCACTAAA | CalFluorOrange560 / BHQ-1 blocked |

| | | |
|---|---|---|
| FAM is Fluorescein. BHQ-1 is Black Hole Quencher-1. BHQ-2 is Black Hole Quencher-2. | | |

## Claims

1. A kit comprising a first sealed container containing a lyophilized polymerase formulation that enables reconstitution into an aqueous polymerase formulation comprising:
at least one polymerase;
α-cyclodextrin at a concentration of from 10 mg/mL to 40 mg/mL;
polysorbate 20 at a concentration of from 0.002% to 0.05% (v/v);
at least one buffering agent; and
a lyoprotectant.

2. The kit of claim 1, wherein the α-cyclodextrin concentration is from 16 mg/mL to 30 mg/mL, from 15 mg/mL to 20 mg/mL, or from 10 mg/mL to 15 mg/mL.

3. The kit of claim 1, wherein the α-cyclodextrin concentration is 20 mg/mL, 17.5 mg/mL, or 12.5 mg/mL.

4. The kit of any one of claims 1 to 3, wherein the polysorbate 20 concentration is from 0.003% to 0.03% (v/v).

5. The kit of any one of claims 1 to 3, wherein the polysorbate 20 concentration is 0.0042% (v/v), 0.0035% (v/v), 0.0026% (v/v), or 0.02% (v/v).

6. The kit of any one of claims 1 to 5, wherein the at least one polymerase is a DNA polymerase.

7. The kit of any one of claims 1 to 6, wherein the lyophilized polymerase formulation further comprises nucleotide triphosphates suitable for performing nucleic acid amplification.

8. The kit of claim 1, wherein the lyoprotectant is trehalose.

9. The kit of claim 8, wherein the trehalose is present at a concentration of from 0.2 M to 0.4 M.

10. The kit of claim 9, wherein the trehalose is present at a concentration of 0.26 M or 0.3 M.

11. The kit of any one of claims 1 to 10, further comprising a second sealed container containing a diluent.

## Patentansprüche

1. Kit, umfassend einen ersten verschlossenen Behälter, der eine lyophilisierte Polymeraseformulierung enthält, die eine Rekonstitution in eine wässrige Polymeraseformulierung ermöglicht, umfassend:
mindestens eine Polymerase;
α-Cyclodextrin in einer Konzentration von 10 mg/ml bis 40 mg/ml;
Polysorbat 20 in einer Konzentration von 0,002 Vol.-% bis 0,05 Vol.-%;
mindestens ein Puffermittel;
und ein Lyoschutzmittel.

2. Kit nach Anspruch 1, wobei die α-Cyclodextrinkonzentration von 16 mg/ml bis 30 mg/ml, von 15 mg/ml bis 20 mg/ml oder von 10 mg/ml bis 15 mg/ml beträgt.

3. Kit nach Anspruch 1, wobei die α-Cyc+lodextrinkonzentration 20 mg/ml, 17,5 mg/ml oder 12,5 mg/ml beträgt.

4. Kit nach einem der Ansprüche 1 bis 3, wobei die Konzentration von Polysorbat 20 von 0,003 Vol.-% bis 0,03 Vol.-% beträgt.

5. Kit nach einem der Ansprüche 1 bis 3, wobei die Konzentration von Polysorbat 20 0,0042 Vol.-%, 0,0035 Vol.-%, 0,0026 Vol.-% oder 0,02 Vol.-% beträgt.

6. Kit nach einem der Ansprüche 1 bis 5, wobei die mindestens eine Polymerase eine DNA-Polymerase ist.

7. Kit nach einem der Ansprüche 1 bis 6, wobei die lyophilisierte Polymerase-Formulierung ferner Nukleotidtriphosphate umfasst, die zur Durchführung einer Nukleinsäureamplifikation geeignet sind.

8. Kit nach Anspruch 1, wobei das Lyoschutzmittel Trehalose ist.

9. Kit nach Anspruch 8, wobei die Trehalose in einer Konzentration von 0,2 M bis 0,4 M vorhanden ist.

10. Kit nach Anspruch 9, wobei die Trehalose in einer Konzentration von 0,26 M bis 0,3 M vorhanden ist.

11. Kit nach einem der Ansprüche 1 bis 10, ferner umfassend einen zweiten versiegelten Behälter, der ein Verdünnungsmittel enthält.

## Revendications

1. Trousse comprenant un premier récipient scellé contenant une formulation de polymérase lyophilisée qui permet une reconstitution en formulation de polymérase aqueuse comprenant :
au moins une polymérase ;
une α-cyclodextrine à une concentration d'environ 10 mg/ml à environ 40 mg/ml ;
du polysorbate 20 à une concentration de 0,002 % à 0,05 % (v/v) ;
au moins un tampon ;
et un lyoprotecteur.

2. Trousse selon la revendication 1, dans laquelle la concentration en α-cyclodextrine est de 16 mg/ml à 30 mg/ml, de 15 mg/ml à 20 mg/ml, ou de 10 mg/ml à 15 mg/ml.

3. Trousse selon la revendication 1, dans laquelle la concentration en α-cyclodextrine est de 20 mg/ml, 17,5 mg/ml ou 12,5 mg/ml.

4. Trousse selon l'une quelconque des revendications 1 à 3, dans laquelle la concentration en polysorbate 20 est de 0,003 % à 0,03 % (v/v).

5. Trousse selon l'une quelconque des revendications 1 à 3, dans laquelle la concentration en polysorbate 20 est de 0,0042 % (v/v), 0,0035 % (v/v), 0,0026 % (v/v), ou 0,02 % (v/v).

6. Trousse selon l'une quelconque des revendications 1 à 5, dans lequel l'au moins une polymérase est une ADN polymérase.

7. Trousse selon l'une quelconque des revendications 1 à 6, dans laquelle la formulation de polymérase lyophilisée comprend en outre des nucléotides triphosphates appropriés pour effectuer une amplification d'acide nucléique.

8. Trousse selon la revendication 1, dans laquelle le lyoprotecteur est le tréhalose.

9. Trousse selon la revendication 8, dans laquelle le tréhalose est présent à une concentration de 0,2 M à 0,4 M.

10. Trousse selon la revendication 9, dans laquelle le tréhalose est présent à une concentration de 0,26 M ou 0,3 M.

11. Trousse selon l'une quelconque des revendications 1 à 10, comprenant en outre un second récipient scellé contenant un diluant.
